# EUROPÄISCHE PATENTSCHRIFT

(11) **EP 2 832 320 B1**
(45) Veröffentlichungstag und Bekanntmachung des Hinweises auf die Patenterteilung: **29.05.2019**
(21) Anmeldenummer: 14176138.7
(22) Anmeldetag: 08.07.2014
(51) Int. Cl.: A61F 2/34, A61F 2/30

(54) **Knochenimplantat mit Andockstellen**
Bone implant with docking sites
Implant osseux ayant des points d'insertion

(30) Priorität: 30.07.2013 DE 102013108160
(43) Veröffentlichungstag der Anmeldung: 04.02.2015
(73) Patentinhaber: AESCULAP AG, 78532 Tuttlingen (DE)
(72) Erfinder: Schneider, Jens, 78315 Radolfzell (DE); Broghammer, Tanja, 78662 Bösingen (DE); Bislimi, Irvin, 78549 Spaichingen (DE); Martin, Patrick, 52074 Aachen (DE)
(74) Vertreter: Winter, Brandl, Fürniss, Hübner, Röss, Kaiser, Polte - Partnerschaft mbB

(56) Entgegenhaltungen:
- EP-A1- 0 554 210
- EP-A1- 2 140 835
- WO-A1-2011/008752
- DE-U1-202011 050 057
- US-A1- 2010 076 572

## Beschreibung

Die vorliegende Erfindung betrifft allgemein ein Knochenimplantat (-modul), vorzugsweise in Form eines Gitterkörpers, mit Andockstellen sowie ein Implantatsystem aus mehreren aneinander zu fixierenden Implantatmodulen, vorzugsweise bestehend aus einer Hüftgelenkpfanne und einem Acetabulum-Augmentat, die mit entsprechend kooperierenden Andockstellen für eine mechanische Haftkopplung versehen sind.

Knochenimplantate der erfindungsgemäßen Gattung dienen allgemein zur Auffüllung von Knochendefekten oder zur Überbrückung und/oder Verbindung von Knochenelementen, wie beispielsweise von zwei Wirbelknochen einer Wirbelsäule. Einige Knochenimplantate, wie beispielsweise künstliche Gelenke, weisen einen Vollkörper auf, um ausreichend hohe Kräfte aufnehmen und in den Knochen weiterleiten zu können. Andere Knochenimplantate haben eine metallische, an der Implantatsoberfläche zumindest partiell an der dem Knochen zugewandten Seite offenporige (offenzellige) Struktur vorzugsweise aufgebaut aus miteinander verbundenen Stegen, sowie weiter vorzugsweise eine Füllung aus Knochenersatzmaterial zur Beschleunigung des Knochendurchbauprozesses nach deren Implantierung in einem Patientenkörper.

Je nach Einsatzzweck können auch diese Implantate (beispielsweise in Form einer so genannten Hüftgelenkpfanne und/oder einer Augmentatskomponente) mit ihrer jeweiligen Gitter- oder Gerüststruktur eine Last tragende Funktion übernehmen, um einen großflächigeren Kontakt mit dem jeweiligen Knochen (beispielsweise im Fall der Hüftgelenkpfanne mit dem Beckenknochen oder im Fall eines sogenannten Cages mit einem Wirbelknochen, etc.) eines Patienten zu schaffen.

Beispielsweise aus der WO 2008/040409 A1 ist ein Gitter - strukturiertes Knochenimplantat beispielhaft in Form einer modularen Augmentatskomponente bekannt, welche trotz der Gitterstruktur eine hohe Lastfähigkeit aufweist sowie ein optimales Einwachsen von Knochenmaterial ermöglicht. Dieses bekannte Knochenimplantat besteht aus einem dreidimensionalen Gerüst aus miteinander verbundenen Stegen, die untereinander verbundene kleine Hohlräume oder Zellen mit einer Größe im Bereich von 0,5 mm bis 6 mm und große Hohlräume in einem Bereich von 6 mm bis 20 mm ausbilden. Sowohl die kleinen wie auch die großen Hohlräume sind angrenzend an die Implantatsoberfläche offenzellig gestaltet, wodurch sich kleine und große Vertiefungen ergeben, die zur Aufnahme von Knochen- oder Knochenersatzmaterial für einen beschleunigten Durchbau des Implantats vorgesehen sind.

Aufgrund der offenzelligen Ausführung des Implantats an dessen Oberfläche entsteht eine von der Gitterstruktur abhängige Oberflächenrauhigkeit, so dass das Implantat auch unmittelbar nach dessen Implantierung einen bestimmten Reibschluss mit dem angrenzenden Knochen eingeht, indem sich die frei vorragenden Stege in das Knochenmaterial verkrallen. Gleiches gilt auch für die Verbindung mit der zugehörigen Hüftgelenkspfanne, die im implantierten Zustand in Folge ihrer ebenfalls offenporigen Teil-Oberfläche reibschlüssig (bzw. mikro-formschlüssig) am Augmentat anliegt und von diesem entsprechend abgestützt wird.

Weiter sei die WO 2011/008752 A1 genannt, in der ein Knochenimplantat-System offenbart ist, das aus zwei halbkugelartigen Schalen besteht, welche wiederum über einen Ring miteinander koppelbar sind. Die untere Schale weist einen ersten Verriegelungsmechanismus auf, der mit Vorsprüngen versehen ist. Die zweite Schale weist einen zweiten Verriegelungsabschnitt auf, der entsprechende Ausnehmungen aufweist, in welche die Vorsprünge eingreifen.

Ferner sei die EP 2 140 835 A1 genannt, aus der konkret ein Acetabulum-Augmentat (Sekundärimplantat) bekannt ist. Dieses bildet einen teilsphärischen Vollkörper, dessen Außenseite eine Knochen-Kontaktfläche bildet und dessen Innenseite an die Form eines Primärimplantats, vorliegend einer Hüftgelenkpfanne angepasst ist. Das bekannte Augmentat hat ebenfalls eine Anzahl von bewusst angeordneten bzw. ausgebildeten Hohlräumen, die zumindest zur Außenseite hin offenzellig sind, um ein Einwachsen des Implantats in das angrenzende Knochenmaterial zu ermöglichen. Ferner ist eine längs geschlitzte Durchgangsöffnung im Augmentat ausgeformt, welche die Außen- und Innenseite des Augmentats miteinander verbindet und die zur Einführung einer Fixierschraube dient.

Die Hüftgelenkpfanne kann somit in die im Wesentlichen konkave Innenseite des Augmentats eingesetzt und zusammen mit dem Augmentat am Beckenknochen des Patienten montiert werden. Hierfür wird zwischen dem modularen Implantatssystem, d.h. zwischen Augmentat und Pfanne ein Knochenzement eingegeben und anschließend das Implantatssystem mittels Schraubenbolzen am Beckenknochen verschraubt. Dabei wird zumindest ein gemeinsamer Schraubenbolzen sowohl durch die Hüftgelenkpfanne und gleichzeitig auch durch die längs geschlitzte Durchgangsöffnung im Augmentat geführt, sodass die Relativlage beider Implantatsteile fixiert bleibt.

Im Stand der Technik besteht bei den bekannten modularen Knochenimplantat - Systemen im Allgemeinen und bei Hüftgelenkpfanne - Augmentat - Systemen im Besonderen das grundsätzliche Problem der mangelhaft andauernden Haftung am Knochen sowie der Aufrechterhaltung der Relativlage insbesondere unmittelbar nach deren Implantation, also zu einem Zeitpunkt, in welchem die Gitterstruktur der Implantatteile noch nicht von Knochengewebe durchbaut ist bzw. der dazwischen eingebrachte Knochenzement noch nicht ausgehärtet ist. Die Aushärtzeit von Knochenzement beträgt in der Regel zwischen 5-10min, während dessen die Relativposition noch nicht stabil ist. In anderen Worten ausgedrückt muss zum Einen während und unmittelbar nach der Implantierung eine ausreichende Haftung zwischen den Implantatelementen untereinander sowie zwischen Implantatelementen und dem Knochen gewährleistet sein, um das Implantatsystem in korrekter Relativlage gleich belasten zu können, ohne dass dieses seine Implantierungsposition verändert, zum Anderen sollte das Implantat möglichst rasch und intensiv durchbaut werden, um schnell seine maximale Belastungsfähigkeit zu erreichen.

Die aus dem Stand der Technik bekannten Verschraubungsmethoden sollten zwar die einzelnen Implantatskomponenten oder Module reibschlüssig aneinander halten, indessen ist die Lagestabilität abhängig von der erreichten Schraubkraft, die je nach Knochenmaterial-Qualität unterschiedlich sein kann. Zur Lösung dieser Problematik wurden daher bereits im Stand der Technik folgende Lösungsansätze vorgeschlagen:
- Gemeinsame Schraubverbindungen beispielsweise gemäß der EP 2 140 835 A1,
- Kopplung von Primär- und Sekundärmodule über daran jeweils ausgebildete Makrostrukturen, z.B. Nuten und Zapfen beispielsweise gemäß der US 6,458,161 B1
- "Stoffschlüssige" Verbindung mittels einer (zusätzlichen) Knochenzement - Zwischenschicht beispielsweise gemäß der EP 2 140 835 A1,

Die bekannten Schraub- und auch Steckverbindungen mit Makrostrukturen erlauben in der Regel nur wenige definierte Relativpositionen zwischen den modularen Implantatskomponenten. Dies wird der individuellen Versorgungssituation der Patienten insbesondere in der Revisionschirurgie nur unzureichend gerecht. Zudem sind viele bekannte Verbindungslösungen nach der Fixierung am Patientenknochen nicht ausreichend mechanisch stabil (beispielsweise bei der Verwendung von Knochenzement) oder benötigen dafür zu viele Verankerungselemente (Schrauben), welche folglich zu viel Raum einnehmen.

Die Anbindung/Kopplung der einzelnen modularen Implantatskomponenten über einen (globalen) Formschluss mit Knochenzement als Zwischenlage könnte zwar die Anforderung nach einer individuellen, variablen Relativpositionierung und auch Positionierung im/am Patientenknochen erfüllen, wodurch auch eine mechanische Ruhe an der Verbindungsstelle zwischen Implantat und Knochen weitestgehend bewirkt wird. Jedoch hat die Verwendung von Knochenzement an sich einige Nachteile:
- die OP-Zeit verlängert sich,
- es ergibt sich eine schwierige Applikation in-situ,
- es entsteht ein Operationszeitdruck aufgrund der erforderlichen Aushärtereaktion,
- ein entsprechender Arbeitsschutz zur/bei der Verarbeitung ist erforderlich,
- ein aufwändiges Säubern nach Einbringung des Knochenzements ist erforderlich,
- Partikel/Bruchstücke können zu Drittkörperverschleiß im Gelenk führen,
- Das Lösen der Verbindung beispielsweise im Fall einer Revision der modularen Implantatskomponenten ist sehr problematisch - ggf. unmöglich.

Angesichts dieser Problematik besteht die Aufgabe der vorliegenden Erfindung darin, ein modulares Knochen - Implantatssystem sowie modulare Implantatskomponenten für dieses System bereit zu stellen, die mit entsprechend kooperierenden Andockstellen für eine mechanische Haftkopplung versehen sind, welche eine möglichst freie relative Positionierbarkeit erlauben. Ein Ziel ist es dabei, die Andockstellen so auszubilden, dass nach Kopplungsschluss Relativbewegungen zwischen den Implantatoberflächen auch unter äußerer Krafteinwirkung verhindert werden. Dabei soll vorzugsweise auf die Verwendung von Knochenzement verzichtet werden können, sodass eine Revision der Implantatskomponenten möglich ist.

Diese Aufgabe sowie die bevorzugten Ziele werden mittels eines Knochen - Implantatsystems bzw. modularer Implantatkomponenten dieses Systems mit den Merkmalen des Anspruchs 1 gelöst. Vorteilhafte Ausgestaltungen der Erfindung sind Gegenstand der Unteransprüche.

Der Grundgedanke der vorliegenden Erfindung beruht im Wesentlichen darauf, zumindest eine modulare Implantatskomponente des Knochen - Implantatsystems an wenigstens einem Teil-Oberflächenabschnitt mit einer engmaschigen (nagelbett- oder filamentartigen) Kopplungsstruktur auszubilden, wohingegen wenigstens eine andere modulare Implantatskomponente des Knochen - Implantatsystems an wenigstens einem Teil-Oberflächenabschnitt mit einer kooperierenden grobmaschigen Kopplungsstruktur (vorzugsweise in Form von wenigstens zwei von der Oberfläche dieser anderen modularen Implantatsoberfläche vorragenden Strukturelemente, insbesondere Verankerungsvorsprüngen wie Stifte, Zapfen, Leisten, etc.) ausgebildet ist, welche mit der engmaschigen (nagelbett- oder filamentartigen) Kopplungsstruktur in Hafteingriff (Form- und/oder Reibschluss) bringbar ist. Entscheidend bei dieser technischen Ausgestaltung ist es demnach, dass die Strukturelemente der einen Kopplungsstruktur (nagelbett- oder filamentartige Struktur) eine Fein - Kopplungsstruktur (Feinrasterung) bilden, wohingegen die Strukturelemente der anderen, korrespondierenden Kopplungsstruktur eine gegenüber der einen Kopplungsstruktur sich ergebende Grob - Kopplungsstruktur (Grobrasterung) darstellen. Auf diese Weise eröffnet die Feinrasterung eine nahezu beliebige Anzahl von Relativ - Kopplungsstellungen und Positionen, wohingegen die Grobrasterung ein flexibles/elastisches Ausweichen der die Feinrasterung bildenden Strukturelemente bei In-Eingriff kommen beider Strukturen erlaubt, sodass die Grob-Strukturelemente quasi zwischen den elastisch auseinander gedrückten Fein-Strukturelementen reibschlüssig eingespannt werden.

In anderen Worten ausgedrückt, sind die Designs der korrespondierenden Oberflächen bzw. Kopplungsstrukturen so gestaltet, dass für deren Ankopplung eine Steck-, Klick-, bzw. Klettverschluss - ähnliche Funktionalität erreicht wird. Bei nahezu beliebig freier Relativ - Positionierung der Oberflächen drücken sich die Struktur- bzw. Verankerungselemente an dem einen Implantatsmodul in/zwischen die Struktur- bzw. Verankerungselemente an dem anderen Implantatsmodul und sorgen dabei durch ein (elastisches) Verklemmen, Verkeilen, Verhaken u.a. für einen stabilen mechanischen Sitz, der mögliche Relativbewegungen in der Grenzfläche zwischen den zu koppelnden Implantatsmodulen verhindert.

Konkreter werden vorzugsweise zwei unterschiedliche Design - Ansätze vorgeschlagen:
- Prinzip "Steck- bzw. Nagelbrett": Hierbei werden im Macrobereich eine Vielzahl von allseits beabstandeten länglichen zapfen-, pin- oder säulenartigen Vorsprüngen / Erhöhungen auf der (Teil-) Oberfläche eines ersten Implantatmoduls an- oder ausgeformt, welche im Fall einer Gitterkörper - Struktur die hierdurch erzielte Mikro - Rauhigkeit der Implantatsoberfläche überlagern. Das daran anzudockende zweite Implantatsmodul des Knochen - Implantatsystems weist ebenfalls eine Anzahl von allseits beabstandeten länglichen zapfen-, pin- oder säulenartigen Vorsprüngen / Erhöhungen auf dessen (Teil-) Oberfläche auf, wobei die Zahl der Vorsprünge kleiner und/oder deren Abstand zueinander größer ist als beim ersten Implantatmodul und ggf. auch deren Dicke (Durchmesser) größer ist als beim ersten Implantatmodul. Dadurch werden die Vorsprünge des ersten Implantatmodul von den dazwischen eingleitenden Vorsprüngen des zweiten Implantatmoduls (flexibel bzw. elastisch) verdrängt, wodurch sich beide Kopplungsstrukturen quasi gegeneinander verspannen.
- Prinzip "Gewebe-, Netz- oder Gitter": In diesem Fall werden eine Vielzahl von längsgestreckten, filamentartigen Strukturen an der Oberfläche des ersten Implantatmoduls ausgeformt, die vorzugsweise miteinander verwebt, vernetzt oder als flexible Gitterstruktur gestaltet sind. Die (Teil-) Oberfläche des zweiten Implantatmoduls weist eine Art Hakenteppich - Struktur (vergleichbar zu einem Klettverbund) auf, die mit der Struktur des ersten Implantatmoduls (lösbar) verhakt.

Wie vorstehend bereits ausgeführt wurde, greifen die wenigstens zwei Verankerungselemente der Grobstruktur des einen Implantatmoduls in, durch die Feinstruktur des anderen Implantatmoduls sich ausbildende Vertiefungen ggf. durch Schnapp- oder Konizitätsgestaltung oder drücken sich in "ausweichendes", elastisch verformbares Netz bzw. Gitter ein. Das aufeinander Pressen führt anschließend zu einem Verklemmen der ineinander greifenden Strukturelemente (Kraftschluss) ggf. unter elastischer Verformung der Strukturelemente der Feinstruktur (Filamente).

Vorzugsweise können Strukturierungen auf/an den Grobstrukturelementen und/oder den Feinstrukturelementen (Filamenten) angeordnet/ausgebildet sein, welche ggf. die Verankerungsstabilität durch Schnapp- oder Verhakwirkung (Formschluss) erhöhen.

Ggf. sind die hervorstehenden Verankerungselemente (Grobstrukturelemente) selbst elastisch verformbar bzw. "herausfahr-/drückbar", z.B. durch einen geeigneten Ausstülpmechanismus, entsprechender Manipulation mit einem chirurgischen Hilfsinstrument und/oder durch Einsetzen einer weiteren Komponente, etwa einem Inlay in das entsprechende Implantatmodul.

Auch ist es denkbar, dass die hervorstehenden Verankerungselemente (Grobstrukturelemente) separate Hilfselemente sind, die zusätzlich in bzw. durch die Oberflächen der Implantatmodule eingeführt werden, z.B. in Form von Nieten, Stiften, Haken, Schrauben, o. ä.

Durch die Ausbildung der einzelnen Knochen-Implantatmodule mit den vorstehend beschriebenen, kooperierenden Kopplungsstrukturen ist es möglich, dass der Anwender die Module (Implantatkomponenten) frei auf-/aneinander positionieren und damit z.B. Knochendefektsituationen exakt adressieren kann. Es gibt keinen zusätzlichen Aufwand oder Risiken durch einen Einsatz von Knochenzement, wie er im Stand der Technik erforderlich ist. D.h., gegenüber der heute üblichen OP-Technik z.B. bei Acetabulum-Augmentaten als ein Implantatmodul kann mit der vorliegenden Erfindung OP-Zeit gespart und das Patientenrisiko minimiert werden. Das Design der co-operierenden Kopplungsstrukturen kann sehr kleinvolumig ausgeführt werden, und ist daher auf viele Implantatsgeometrien übertragbar. Schließlich können für den Fall, dass ggf. geringfügige Relativbewegungen der miteinander gekoppelten Module auftreten sollten, diese durch das elastische Materialverhalten der Strukturelemente zumindest der Feinstruktur ausgeglichen werden. Es besteht somit kein Risiko von Knochenzement-Abrieb und/oder sekundärem Drittkörperverschleiß.

Für eine erfindungsgemäße Kopplung geeignete Implantatmodule wäre beispielsweise
- Augmentat an Hüftgelenkpfanne,
- Inlays in Hüftgelenkpfannen oder Rekonstruktionsschalen,
- (proximaler) Mantel bzw. Defektaugmentate an Hüftschäften,
- Augmentate an Knieprothesen,
- Wirbelsäulen-Implantate, z.B. modularer Aufbau von sogenannten cages.

Gemäß einem anderen oder zusätzlichen Aspekt der Erfindung können die Strukturelemente der Fein- und/oder Grobstruktur zum übrigen (Gerüst-)material des Implantatmoduls unterschiedlich sein oder zumindest eine diesbezüglich unterschiedliche Materialeigenschaft aufweisen. In diesem Fall kann anstelle oder zusätzlich zur besonderen Formgebung oder Elementabstand auch die Materialwahl für die Strukturelemente dazu genutzt werden, bestimmte Friktionseigenschaften zu erhalten.

Vorteilhaft ist es, wenn das erfindungsgemäße Knochenimplantat (Gitterkörper) nach einem additiven bzw. generativen Verfahren, wie z.B. dem Lasersintern aus einem hierfür geeigneten Material hergestellt ist.

Ein anderer Aspekt der Erfindung richtet sich auf die Bereitstellung eines Hüftgelenkimplantats bestehend aus einer Hüftgelenkpfanne und einem hierzu separaten Knochenimplantat gemäß einem der vorstehenden Aspekte als Acetabulum-Augmentat. Dieses Augmentat ist auf die Hüftgelenkpfanne in der Weise abgestimmt, als dass die Kopplungsstruktur des Augmentats ausschließlich auf der dem Hüftgelenk zugewandten konkaven Seitenoberfläche ausgebildet ist. Damit wird ein planes Anliegen der Hüftgelenkpfanne am Augmentat bei gleichzeitig optimierter Friktions-/Formschlusseigenschaft zwischen den beiden Modulen erreicht.

Vorteilhaft ist es hierbei, wenn (auch) die konkave Seitenoberfläche des Augmentats und/oder die korrespondierende konvexe Seitenoberfläche der Hüftgelenkpfanne durch die Gitterstruktur des jeweiligen Implantatmodul - Körpers offenporig ist, sodass zwischen der Hüftgelenkpfanne und dem Augmentat ein vorbestimmter Reibschluss zusätzlich zu dem Haftschluss durch die Kopplungsstrukturen erhalten wird.

Die Erfindung wird nachstehend anhand bevorzugter Ausführungsbeispiele unter Bezugnahme auf die begleitenden Figuren näher erläutert.

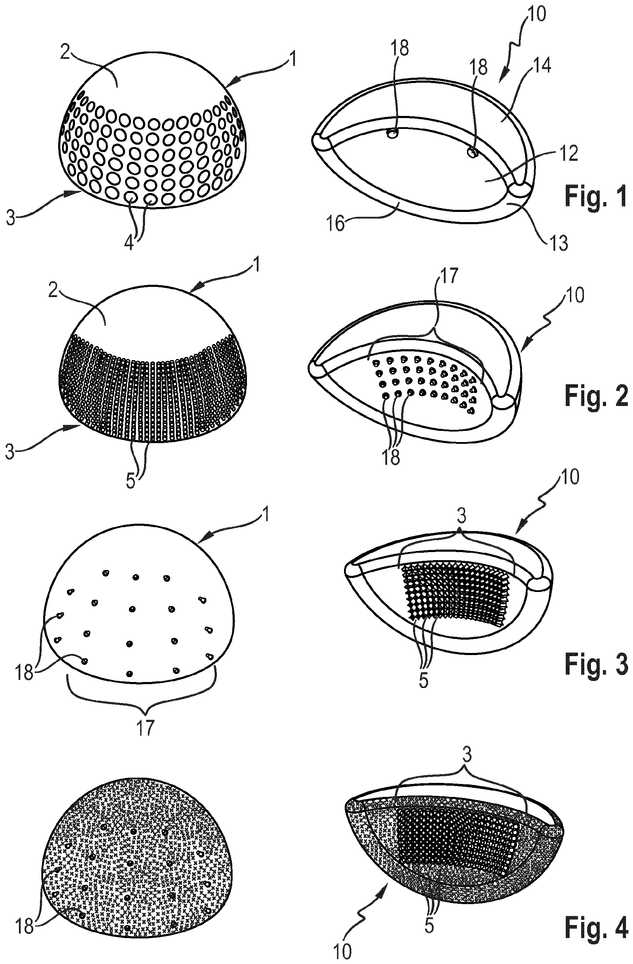

Fig. 1 zeigt in einer Perspektivenansicht die konvexe Seite eines ersten Implantatmoduls vorzugsweise in Form einer vollmateriellen Hüftgelenkpfanne mit einer daran/darauf ausgebildeten feinen Kopplungsstruktur sowie die konkave Außenseite eines zweiten Knochenimplantatmoduls in der Form eines Augmentats, vorzugsweise eines vollmateriellen Acetabulum-Augmentats mit einer daran/darauf ausgebildeten (kooperierenden) groben Kopplungsstruktur zur Bereitstellung eines (haftkoppelbaren) Knochenimplantat - Systems gemäß einem ersten bevorzugten Ausführungsbeispiel der Erfindung,

Fig. 2 zeigt in einer Perspektivenansicht die konvexe Seite eines ersten Implantatmoduls vorzugsweise in Form einer vollmateriellen Hüftgelenkpfanne mit einer daran/darauf ausgebildeten feinen Kopplungsstruktur sowie die konkave Außenseite eines zweiten Knochenimplantatmoduls in der Form eines Augmentats, vorzugsweise eines vollmateriellen Acetabulum-Augmentats mit einer daran/darauf ausgebildeten (kooperierenden) groben Kopplungsstruktur zur Bereitstellung eines (haftkoppelbaren) Knochenimplantat - Systems gemäß einem zweiten bevorzugten Ausführungsbeispiel der Erfindung,

Fig. 3 zeigt in einer Perspektivenansicht die konvexe Seite eines ersten Implantatmoduls vorzugsweise in Form einer vollmateriellen Hüftgelenkpfanne mit einer daran/darauf ausgebildeten groben Kopplungsstruktur sowie die konkave Außenseite eines zweiten Knochenimplantatmoduls in der Form eines Augmentats, vorzugsweise eines vollmateriellen Acetabulum-Augmentats mit einer daran/darauf ausgebildeten (kooperierenden) feinen Kopplungsstruktur zur Bereitstellung eines (haftkoppelbaren) Knochenimplantat - Systems gemäß einem dritten bevorzugten Ausführungsbeispiel der Erfindung,

Fig. 4 zeigt in einer Perspektivenansicht die konvexe Seite eines ersten Implantatmoduls vorzugsweise in Form einer Gitter - aufgebauten Hüftgelenkpfanne mit einer daran/darauf ausgebildeten groben Kopplungsstruktur sowie die konkave Außenseite eines zweiten Knochenimplantatmoduls in der Form eines Augmentats, vorzugsweise eines Gitter - aufgebauten Acetabulum-Augmentats mit einer daran/darauf ausgebildeten (kooperierenden) feinen Kopplungsstruktur zur Bereitstellung eines (haftkoppelbaren) Knochenimplantat - Systems gemäß einem vierten bevorzugten Ausführungsbeispiel der Erfindung,

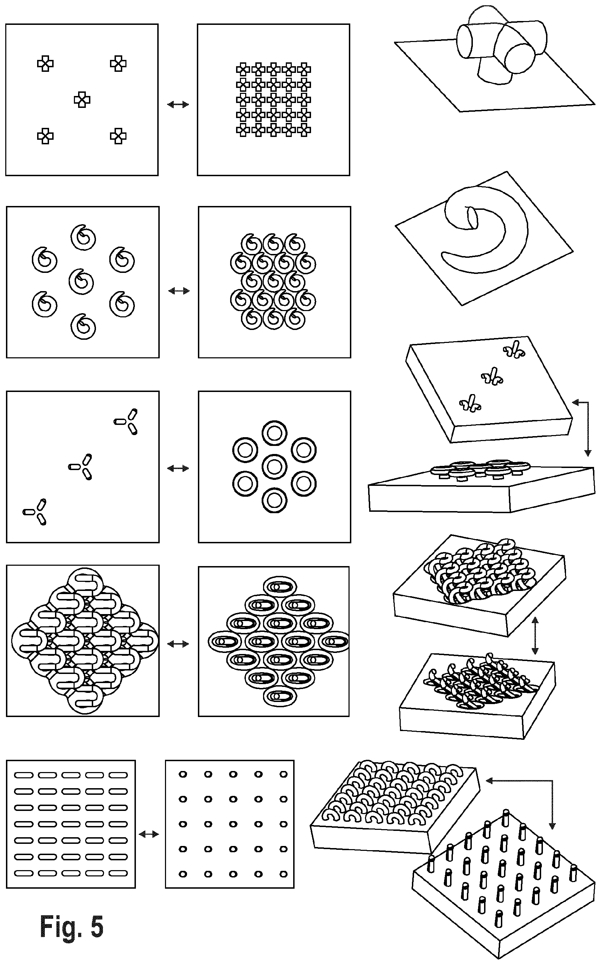

Fig. 5 zeigt eine erste Tabelle mit möglichen kooperierenden Kopplungselementen einer feinen und einer groben Kopplungsstruktur zur Ausbildung einer elastischen Kopplung/Verbindung,

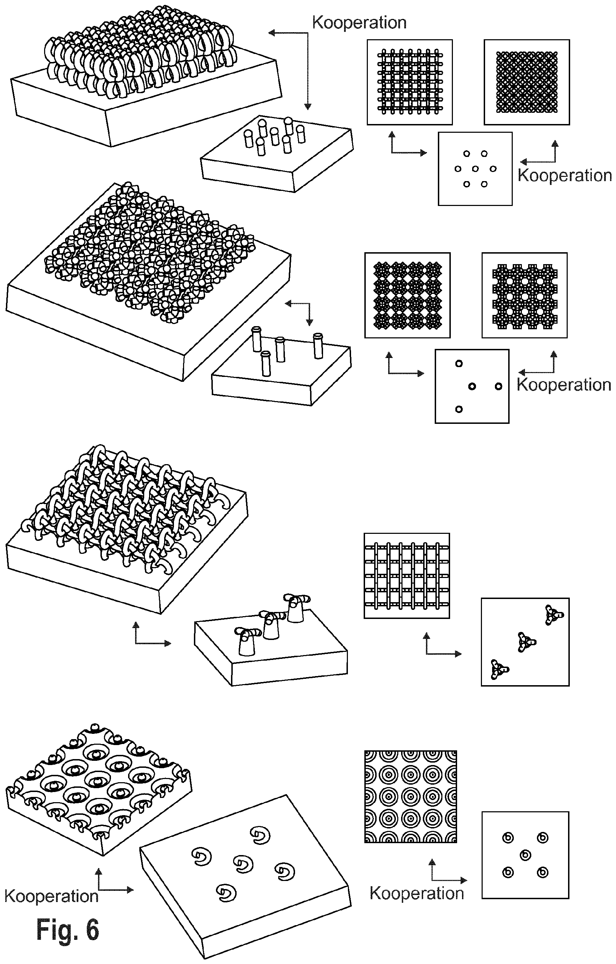

Fig. 6 zeigt eine zweite Tabelle mit möglichen kooperierenden Kopplungselementen einer feinen und einer groben Kopplungsstruktur zur Ausbildung einer elastisch-festen Kopplung/Verbindung,

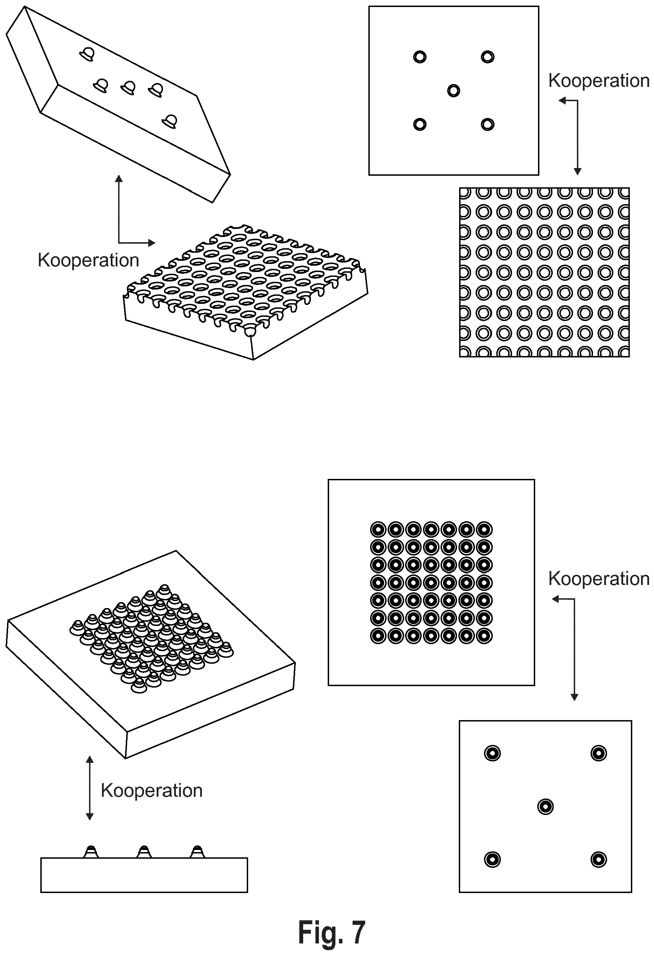

Fig. 7 zeigt eine dritte Tabelle mit möglichen kooperierenden Kopplungselementen einer feinen und einer groben Kopplungsstruktur zur Ausbildung einer festen Kopplung/Verbindung und

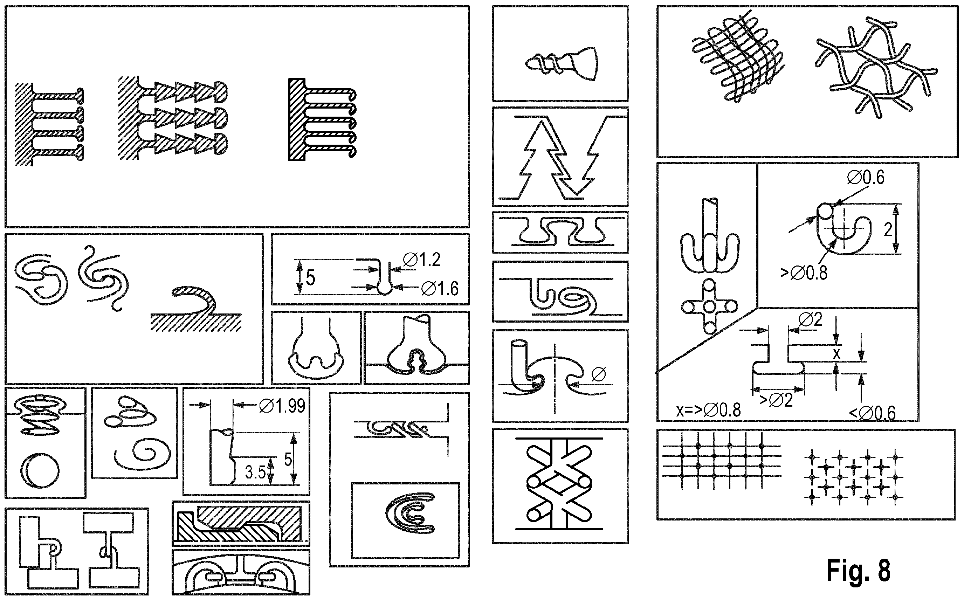

Fig. 8 zeigt einen Auszug unterschiedlicher Kopplungselement-Designs zur Dokumentation der Vielzahl an Gestaltungs- und/oder Kombinationsmöglichkeiten kooperierender Kopplungsstrukturen.

Das in der Fig. 1 dargestellte erste Knochenimplantatmodul (Vollkörper) 1, ist gemäß einem bevorzugten Ausführungsbeispiel der vorliegenden Erfindung in Form einer Hüftgelenkpfanne vorgesehen, wobei auch andersartige Knochenimplantate wie Wirbelknochen - Cages, Hüftgelenkschäfte, etc. vorstellbar sind.

Die vorliegende Hüftgelenkpfanne hat eine (nicht weiter dargestellte) konkave Innenseite zur schwenkbaren Aufnahme eines Hüftgelenks sowie eine konvexe Außenseite 2 zur fixen Verbindung mit einem Beckenknochen und nimmt so im wesentlichen die Form einer Halbschale an. An der konvexen Außenseite 2 ist zumindest in einem teilweisen Oberflächenabschnitt eine feine Kopplungsstruktur 3 ausgebildet. Diese besteht gemäß dem ersten Ausführungsbeispiel aus einer Vielzahl von Strukturelementen 4 vorzugsweise in Form von Sacklöchern, die schachbrettartig über einen Teilumfang der Hüftgelenkpfanne 1 (d.h. vorzugsweise symmetrisch) angeordnet sind.

Das in der Fig. 1 dargestellte zweite Knochenimplantatmodul (Vollkörper) 10 vorliegend in der beispielhaften Form eines mit der Hüftgelenkpfanne 1 wirkverbindenden Acetabulum - Augmentats gemäß der Erfindung hat im Wesentlichen ein teilsphärisches Volumen mit einer konkaven Innenseite 12 und einer konvexen Außenseite 13, deren jeweilige Mittelpunkte (nicht dargestellt) voneinander beabstandet sind. Auf diese Weise entsteht eine ebene (plane) Fläche 14 an einer die konkave und konvexe Seite 12, 13 miteinander verbindenden Stirnseite, wohingegen auf der hierzu gegenüberliegenden Stirnseite eine im Wesentlichen scharfe Übergangskante 16 zwischen der konkaven und konvexen Seite 12, 13 ausgebildet wird. Auf/an der konkaven Innenseite 12 ist zumindest über einen Teil - Oberflächenabschnitt des Acetabulum - Augmentats eine grobe Kopplungsstruktur 17 vorgesehen/ausgebildet, welche mit der feinen Kopplungsstruktur 3 der Hüftgelenkpfanne 1 kooperiert, derart, dass beide Strukturen 3, 17 miteinander in Eingriff bringbar sind, um die Knochenimplantatsmodule 1, 10 kraft- und/oder formschlüssig haftzuverbinden.

Im Konkreten weist die grobe Kopplungsstruktur 17 eine gegenüber der feinen Kopplungsstruktur 3 verringerte Anzahl (mindestens zwei) von Strukturelementen 18 vorzugsweise in Form von zapfen- oder stiftartigen Vorsprüngen auf, die in einem gegenüber den Sacklöchern 4 größerem Abstand zueinander angeordnet sind. Die Größe und Form der Vorsprünge 18 sind so gewählt, dass sie in die Sacklöcher 4 reibschlüssig eingepresst werden können.

Um demnach das Acetabulum - Augmentat 10 mit der Hüftgelenkpfanne 1 fest zu koppeln, braucht das Augmentat 10 lediglich in einer gewählten Relativausrichtung an die Hüftgelenkpfanne 3 angelegt und anschließend angedrückt zu werden, wobei die Vorsprünge 18 in entsprechende Sacklöcher 4 reibschlüssig eingreifen. Dabei sei an dieser Stelle darauf hingewiesen, dass die beiden Kopplungsstrukturen 3, 17 nicht auf die jeweils genannten Module beschränkt sind sondern unter den Modulen beliebig vertauscht sein können.

Das erste Ausführungsbeispiel bietet somit auf einfache Weise unter geringem Herstellungsaufwand die Möglichkeit, eine feste Verbindung zwischen den beiden Knochenimplantat - Modulen 1, 10 des erfindungsgemäßen Implantatsystems bereit zu stellen ohne die Notwendigkeit der Zwischenfügung von Knochenzement oder einer (gemeinsamen) Fixierschraube, wobei letztere natürlich optional zusätzlich so vorgesehen sein kann, wie dies aus dem genannten Stand der Technik bekannt ist und daher an dieser Stelle nicht weiter beschrieben werden muss.

Jedoch hat sich gezeigt, dass die Bereitstellung der Kopplungsstrukturen 3, 17 gemäß der vorstehenden Beschreibung eine nur kleine Anzahl von Relativpositionen erlaubt. Daher sieht das zweite Ausführungsbeispiel der vorliegenden Erfindung eine vom ersten Ausführungsbeispiel abweichende Konstruktion für die grobe und feine Kopplungsstruktur 3, 17 vor.

Gemäß dem zweiten Ausführungsbeispiel besteht auch die feine Kopplungsstruktur 3 auf Seiten der Hüftgelenkpfanne 1 aus einer Vielzahl von stift- oder nagelförmigen Vorsprüngen (Filamente) 5, die nach Art eines Nagelbretts dicht (engmaschig) aneinander stehen und somit zwischen sich eine Vielzahl von Einstecktaschen definieren. Die Vorsprünge 5 sind dabei dünn, bzw. mit geringer Materialstärke ausgebildet und daher elastisch biegsam bzw. biegeflexibel. Die grobe Kopplungsstruktur 17 am Acetabulum - Augmentat 10 besteht, wie im ersten bevorzugten Ausführungsbeispiel der Erfindung, aus einer gegenüber der feinen Kopplungsstruktur 3 verringerten Anzahl von zapfen- oder stegförmigen Vorsprüngen 18, die einen gegenüber den Stiften 5 weiteren Zwischenabstand haben und auch eine größere Materialstärke aufweisen. Sie sind daher bezüglich der Stifte 5 nicht für eine Biegung vorgesehen sondern vergleichsweise starr ausgebildet. Des Weiteren sind gemäß dem zweiten Ausführungsbeispiel optional mehr als zwei Zapfen 18 vorgesehen, welche schachbrettartig am betreffenden Knochenimplantat - Modul 10 des Knochenimplantat - Systems angeordnet sind.

Zur festen Verbindung beider Module 1, 10 werden diese ebenfalls aufeinander gedrückt, wobei die Zapfen 18 der Grob-Kopplungsstruktur 17 in die Aufnahmetaschen zwischen den Stiften 5 der Fein-Kopplungsstruktur 3 reibschlüssig eindringen. Da die Zwischenabstände der Zapfen 18 größer sind als die Zwischenabstände der Stifte 5 und auch die Materialstärken der Zapfen 18 größer sind als die Materialstärken der Stifte 5, werden die Stifte 5 unter Einwirkung der Zapfen 18 zur Seite gebogen und weichen so in Richtung hin zu dem nicht besetzten Aufnahmetaschen aus. Dadurch werden die Zapfen 18 quasi zwischen den Stiften 5 (elastisch) eingespannt und fest darin gehalten. Diese Haftwirkung lässt sich verstärken, indem die Zapfen 18 und/oder die Stifte 5 mit Profilen ausgeformt sind (z.B. Hakenförmig) und/oder eine Oberflächestruktur aufweisen, wodurch sich beide Strukturelemente 5, 18 zusätzlich zur Verklemmung auch (formschlüssig) verhaken.

Durch die Ausbildung der feinen Kopplungsstruktur 3 vorliegend in Form eines Nagelbretts wird eine nahezu beliebe Anzahl von Relativpositionen zwischen den beiden Modulen 1, 10 bereitgestellt. Dabei sei darauf hingewiesen, dass das Nagelbrett-Design der feinen Kopplungsstruktur 3 nur beispielhaft ist und auch beispielsweise durch ein engmaschiges Gitter, Geflecht oder eine Schlingenstruktur ersetzt sein kann. Auch ist die Zuordnung der Fein- und Grobstruktur 3, 17 auf die genannten Module nicht beschränkt sondern im Wesentlichen beliebig, wie dies in der Fig. 3 dargestellt ist.

Demnach ist die Feinstruktur 3 an der konkaven Innenseite 12 des Acetabulum - Augmentats 10 und die Grobstruktur 18 an der konvexen Außenseite 2 der Hüftgelenkpfanne 1 vorgesehen. Ferner ist die Ausbildung der einzelnen Module als Vollmaterialkörper nicht notwendig. Vielmehr ist es auch möglich, alle oder ausgewählte Module mit einer Gitterstruktur auszubilden, wie dies in der Fig. 4 angedeutet ist.

Demzufolge ist das Acetabulum - Augmentat 10 und/oder die Hüftgelenkpfanne 1 mit jeweils der vorstehend beschriebenen äußeren Form durch ein dreidimensionales Gerüst bzw. eine Gitterstruktur gebildet, bestehend aus miteinander verbundenen Stegen unter Ausbildung eines vorzugsweise sich stetig wiederholenden Gittermusters. Das Gittermuster kann eine Art Diamantgitterstruktur haben, die jeweils einen Hohlraum einschließt, der nach allen Richtungen offen ist. Werden diese Muster dreidimensional aneinandergefügt, ergibt sich die vorstehend genannte Gitterstruktur bestehend aus einer Vielzahl von Stegen und Hohlräumen, die zwischen den Stegen hindurch miteinander verbunden sind, wie dies in der Fig. 4 angedeutet wird.

Wie aus der Fig. 4 weiter zu erkennen ist, ist die Hüftgelenkpfanne 1 wie auch das Augmentat 10 jeweils als zumindest teilweise offenporiger bzw. offenzelliger Gegenstand (Volumen) ausgebildet. In anderen Worten ausgedrückt, reicht im vorliegenden Ausführungsbeispiel die Gitterstruktur bis an die jeweils konkave und konvexe Oberfläche, derart, dass die daran angrenzenden Hohlräume vorliegend sämtlich offenzellig sind und die daran angrenzenden Stege nach außen vorragen. Dabei sei darauf hingewiesen, dass in Abhängigkeit von der geforderten Festigkeit und/oder Steifigkeit des jeweiligen Implantatmoduls 1, 10 die genanten Oberflächen auch zumindest teilweise geschlossen, d.h. mit einem Vollmaterialmantel überzogen sein können.

Durch die offenzellige Ausführung gemäß der Fig. 4 ergibt sich eine raue, sowie poröse (Teil-) Oberfläche der Implantatmodule 1, 10, sodass das erste Implantatmodul (z.B. Hüftgelenkpfanne) 1 in/an dem zweiten Implantatmodul (z.B. Acetabulum - Augmentat) 10 zusätzlich zu den vorstehen beschriebenen Kopplungsmechanismen in Reibkontakt kommt, wobei der hierbei erzeugte Kraftschluss durch die an der Oberfläche freiliegenden/vorragenden Stege einem Relativverrutschen der Implantatmodule 1, 10 entgegenwirken kann. Gleichzeitig schafft die Offenzelligkeit der an die Oberfläche angrenzenden Hohlräume die Möglichkeit eines allmählichen Durchbaus der Implantatmodule 1, 10 mit Knochenmaterial, wobei dieser Vorgang durch ein vorheriges (zumindest teilweises) Befüllen der Hohlräume beispielsweise mit Knochenersatzmaterial beschleunigbar ist.

An dieser Stelle sei darauf hingewiesen, dass die Kopplungsstrukturen 3 und/oder 17 stoffeinstückig mit den jeweiligen Implantatsmodulen 1, 10 etwa durch Lasersintern hergestellt sein können oder durch separate Strukturelemente 5, 18 gebildet sein können, beispielsweise durch Einsteckstifte oder Einsteckzapfen, welche in vorgebildete Bohrungen am jeweiligen Modul einsteckbar sind oder durch Strukturelementmatten /-platten, welche auf die (Teil-) Oberflächen der einzelnen Module aufgebracht (aufgeklebt) werden können.

Nachfolgend werden anhand der Fig. 5 bis 8 unterschiedliche Designs sowie Kombinationsmöglichkeiten von Strukturelementen der feinen und groben Kopplungsstrukturen auszugsweise beschrieben, welche sämtlich unter den Erfindungsgedanken gemäß dem anliegenden Anspruchssatz fallen. Dabei sei ausdrücklich darauf hingewiesen, dass auch weitere (nicht gezeigte) Formen für die Strukturelemente vorgesehen sein können, sofern diese eine Vielzahl von Relativpositionen der zu koppelnden Implantatsmodule erlauben und eine ggf. wieder lösbare Kopplung mit ausreichend hoher Kopplungskraft ergeben.

In der Fig. 5 ist eine Anzahl von verschiedenen kooperationsfähigen Strukturelement-Konstruktionen tabellarisch dargestellt, welche jeweils elastische Eigenschaften aufweisen und damit eine im Wesentlichen elastische Kopplung ergeben.

Beispielsweise können die Strukturelemente der beiden kooperierenden Kopplungsstrukturen pin-windrad-förmig, oder spiral-/hakenförmig mit einer konisch sich verjüngenden Erstreckung ausgeformt sein, wobei sich zwischen der feinen und der groben Kopplungsstruktur lediglich die Abstände unter den Strukturelementen unterscheiden. Alternativ hierzu wäre es auch denkbar die eine (grobe) Kopplungsstruktur mit hakenförmigen Strukturelementen und die kooperierende andere (feine) Kopplungsstruktur mit pin-windrad-förmigen, pin-kugel-förmigen oder noppenförmigen Strukturelementen auszubilden.

Gemäß der Fig. 6 kann es vorgesehen sein, die feine Kopplungsstruktur mit Strukturelementen in Form von Sacklöchern oder zurückgesetzten Pins auszubilden, und die Strukturelemente der groben Kopplungsstruktur als spiral-/hakenförmig mit einer konisch sich verjüngenden Erstreckung ausgeformte Vorsprünge vorzusehen, welche in die Sacklöcher bzw. Sacklöcher mit zentralen (zurückgesetzten) Pins eingepresst werden können. Dadurch ergibt sich eine Kooperation zwischen elastische/flexiblen Strukturelementen mit starren Strukturelementen. Ähnliches wäre aber auch erreichbar, indem die Kopplungselemente der einen (feinen) Kopplungsstruktur eine Art Zellstruktur (kristallartig oder verwoben/verkettet) aufweisen und damit starr sind, wohingegen die Strukturelemente der kooperierenden anderen Kopplungsstruktur beispielsweise als Pins mit Kugel, Windrad oder Teller ausgebildet sind.

In Fig. 7 sind Beispiele gezeigt, wonach die Strukturelemente kegel- oder kugelförmige Vorsprünge oder Sacklöcher sein können. Dabei können die die kegel- oder kugelförmigen Vorsprünge nach Art eines Nagelbretts jeweils miteinander kooperieren oder mit den Sacklöchern gekoppelt werden. In beiden Fällen ergäbe sich eine im Wesentlichen starre Kopplung.

Die Fig. 8 zeigt eine Vielzahl weiterer Formen für kooperierende Strukturelemente beispielsweise als gegeneinander verhakende Tannenformen, Schrauben- oder Wendelformen, Haken- und Ösenformen, Ankerformen, Gitterstreben, Polypenformen, etc. welche sämtlich Hinterscheidungen ausbilden und daher ein gegenseitiges Verhaken gewährleisten.

Zusammenfassend wird ein Knochenimplantatsystem offenbart mit einer Anzahl von miteinander fest koppelbaren Knochenimplantatmodulen, die jeweils mit zumindest einem Oberflächenabschnitt versehen sind, der für ein In Kontakt kommen mit einem anderen Knochenimplantatmodul in einer wählbaren Relativlage vorgesehen ist. Erfindungsgemäß ist der zumindest eine Oberflächenabschnitt eines ersten Knochenimplantatmoduls mit einer feinen Kopplungsstruktur versehen oder ausgebildet bestehend aus einer Vielzahl von engmaschig angeordneten Strukturelementen und der zumindest eine Oberflächenabschnitt eines zweiten Knochenimplantatmoduls ist mit einer kooperierenden groben Kopplungsstruktur versehen oder ausgebildet bestehend aus einer gegenüber der feinen Kopplungsstruktur geringeren Anzahl von weitmaschig angeordneten Strukturelementen.

## Patentansprüche

1. Knochenimplantatsystem mit einer Anzahl von miteinander fest koppelbaren Knochenimplantatmodulen (1, 10), die jeweils mit zumindest einem Oberflächenabschnitt versehen sind, die für ein miteinander in Kontakt kommen bei Kopplung mindestens zweier Knochenimplantatmodule in einer wählbaren Relativlage vorgesehen sind, wobei der zumindest eine Oberflächenabschnitt zumindest eines ersten Knochenimplantatmoduls (1) mit einer feinen Kopplungsstruktur (3) versehen oder ausgebildet ist bestehend aus einer Vielzahl von engmaschig angeordneten Strukturelementen (4, 5) und der zumindest eine Oberflächenabschnitt zumindest eines zweiten Knochenimplantatmoduls (10) mit einer kooperierenden groben Kopplungsstruktur (17) versehen oder ausgebildet ist bestehend aus einer gegenüber der feinen Kopplungsstruktur (3) geringeren Anzahl von weitmaschig angeordneten Strukturelementen (18), **dadurch gekennzeichnet, dass**
die Strukturelemente (4, 5) der feinen Kopplungsstruktur (3) eine Vielzahl von engmaschig beabstandeten Öffnungen oder Aufnahmetaschen bilden und die Strukturelemente (18) der groben Kopplungsstruktur (17) aus wenigstens zwei weitmaschig beabstandeten Vorsprüngen bestehen, welche für ein reibschlüssiges Eindringen in ausgewählte/auswählbare Öffnungen oder Aufnahmetaschen der feinen Kopplungsstruktur (3) angepasst sind;
die Aufnahmetaschen durch eine Vielzahl von stiftförmigen, biegeflexiblen Vorsprüngen (5), ein engmaschiges, flexibles Oberflächengitter oder ein flexibles Schlingenprofil an der Oberfläche des ersten Implantatmoduls (1) gebildet sind, welche jeweils die Strukturelemente der feinen Kopplungsstruktur (3) darstellen; und
die wenigstens zwei Vorsprünge (18) der groben Kopplungsstruktur (17) gegenüber den Strukturelementen (5) der feinen Kopplungsstruktur (3) biegestarr sowie größer/weiter dimensioniert sind als die von den Strukturelementen (5) der feinen Kopplungsstruktur (3) ausgebildeten Aufnahmetaschen, um bei Eindringen in ausgewählte Aufnahmetaschen ein flexibles Auseinanderdrücken der Strukturelemente (5) der feinen Kopplungsstruktur zu bewirken.

2. Knochenimplantatsystem nach Anspruch 1, **dadurch gekennzeichnet, dass** die Strukturelemente (4, 5, 18) der beiden kooperierenden Kopplungsstrukturen (3, 17) in Reib- und Formschlusseingriff bringbar sind.

3. Knochenimplantatsystem nach Anspruch 1, **dadurch gekennzeichnet, dass** die Öffnungen (4) durch Sacklöcher an der Oberfläche des ersten Knochenimplantatmoduls (1) gebildet sind.

4. Knochenimplantat nach einem der vorstehenden Ansprüche, **dadurch gekennzeichnet, dass** das eine Implantatmodul (10) ein Augmentat und vorzugsweise ein Acetabulum-Augmentat und das andere Implantatmodul (1) vorzugsweise eine Hüftgelenkpfanne ist.

5. Knochenimplantatsystem nach einem der vorstehenden Ansprüche, **dadurch gekennzeichnet, dass** zumindest ein, vorzugsweise alle Knochenimplantatmodule einen Grundkörper mit Gitterstruktur haben, der dadurch an seiner Oberfläche zumindest teilbereichsweise offenporig wird, wodurch sich zusätzlich zu den Kopplungsstrukturen zwischen zwei miteinander gekoppelten Knochenimplantatmodulen ein Oberflächen - Reibschlusskontakt ausbildet.

6. Knochenimplantatsystem nach einem der vorstehenden Ansprüche, **dadurch gekennzeichnet, dass** die Kopplungsstrukturen (3, 17) stoffeinstückig mit dem Implantatmodul vorzugsweise durch ein Laser-Sinterverfahren oder separat vorzugsweise durch separate Stifte oder Gittermatten bereitstellbar sind.

7. Knochenimplantatsystem nach einem der vorstehenden Ansprüche, **dadurch gekennzeichnet, dass** das Material, aus welchem die Strukturelemente (5, 18) der Kopplungsstrukturen (3, 17) gefertigt sind, zum übrigen Implantatmodul - Material des Knochenimplantats (1, 10) unterschiedlich ist oder zumindest eine unterschiedliche Materialeigenschaft vorzugsweise eine höhere Flexibilität hat.

## Claims

1. Bone implant system having a number of bone implant modules (1, 10) which can be securely coupled with one another, which are respectively equipped with at least one surface section, which in order to come into contact with each other in the case of the coupling of at least two bone implant modules are provided in a selectable relative position, wherein the at least one surface section of at least one first bone implant module (1) is provided or formed with a fine coupling structure (3) comprising a multiplicity of structural elements (4, 5) disposed in a fine-meshed manner and the at least one surface section of at least one second bone implant module (10) is provided or formed with a cooperating rough coupling structure (17) comprising a number, smaller than in the case of the fine coupling structure (3), of structural elements (18) disposed in a broad-meshed manner, **characterised in that**
the structural elements (4, 5) of the fine coupling structure (3) form a multiplicity of openings or receiving pockets spaced in a close-meshed manner and the structural elements (18) of the rough coupling structure (17) comprise at least two protuberances spaced in a wide-meshed manner, which are adapted for a friction-fitting penetration into selected/selectable openings or receiving pockets of the fine coupling structure (3);
the receiving pockets are formed by a multiplicity of pin-shaped, bendably flexible protuberances (5), a close-meshed, flexible surface grid or a flexible loop profile on the surface of the first implant module (1), which respectively constitute the structural elements of the fine coupling structure (3); and
the at least two protuberances (18) of the rough coupling structure (17) are flexurally resistant with respect to the structural elements (5) of the fine coupling structure (3) and are larger/broader in size than the receiving pockets formed by the structural elements (5) of the fine coupling structure (3), in order at the penetration into selected receiving pockets to achieve that the structural elements (5) of the fine coupling structure are pushed apart in a flexible manner.

2. Bone implant system according to claim 1, **characterised in that** the structural elements (4, 5, 18) of the two cooperating coupling structures (3, 17) can be brought into friction-fitting and form-fitting engagement.

3. Bone implant system according to claim 1, **characterised in that** the openings (4) are formed by blind holes on the surface of the first bone implant module (1).

4. Bone implant system according to any of the preceding claims, **characterised in that** the one implant module (10) is an augmentate and preferably an acetabulum augmentate and the other implant module (1) is preferably an acetabulum.

5. Bone implant system according to any of the preceding claims, **characterised in that** at least one, preferably all bone implant modules have a basic body having a grid structure, which thereby becomes at least partially regionally open-pored, as a result of which, in addition to the coupling structures between two bone implant modules coupled with one another, a surface frictional contact forms.

6. Bone implant system according to any of the preceding claims, **characterised in that** the coupling structures (3, 17) can be provided materially-integral with the implant module preferably by means of a laser-sintering method or separately preferably by means of separate pins or grid mats.

7. Bone implant system according to any of the preceding claims, **characterised in that** the material from which the structural elements (5, 18) of the coupling structures (3, 17) are made, is different from the remaining implant module material of the bone implant (1, 10) or has at least one different material characteristic, preferably a higher flexibility.

## Revendications

1. Système d'implant osseux avec un nombre de modules d'implant osseux (1, 10) pouvant être couplés solidement l'un à l'autre, qui sont pourvus respectivement d'au moins une section de surface, qui sont prévues pour entrer en contact l'une avec l'autre lors du couplage d'au moins deux modules d'implant osseux dans une position relative sélectionnable, dans lequel l'au moins une section de surface d'au moins un premier module d'implant osseux (1) est dotée de ou réalisée avec une structure de couplage fine (3) composée d'une pluralité d'éléments structuraux (4, 5) agencés en un maillage serré et l'au moins une section de surface d'au moins un second module d'implant osseux (10) est dotée de ou réalisée avec une structure de couplage grossière coopérante (17) composée d'un nombre d'éléments structurels (18) agencés en maillage large inférieur à celui-ci de la structure de couplage fine (3), **caractérisé en ce que**
les éléments structurels (4, 5) de la structure de couplage fine (3) forment une pluralité d'ouvertures ou poches de réception espacées en maillage serré et les éléments structurels (18) de la structure de couplage grossière (17) se composent d'au moins deux saillies espacées en maillage large, lesquelles sont adaptées pour une pénétration par frottement dans des ouvertures ou poches de réception sélectionnées/sélectionnables de la structure de couplage fine (3) ;
les poches de réception sont formées par une pluralité de saillies (5) flexibles en forme de tige, une grille de surface flexible en maillage serré ou un profil de lacet flexible au niveau de la surface du premier module d'implant (1), lesquelles représentent respectivement les éléments structurels de la structure de couplage fine (3) ; et
les au moins deux saillies (18) de la structure de couplage grossière (17) sont rigides à la flexion par rapport aux éléments structurels (5) de la structure de couplage fine (3) ainsi que dimensionnées plus grandes/plus larges que les poches de réception réalisées par les éléments structurels (5) de la structure de couplage fine (3), pour entraîner lors de la pénétration dans des poches de réception sélectionnées un écartement flexible des éléments structurels (5) de la structure de couplage fine.

2. Système d'implant osseux selon la revendication 1, **caractérisé en ce que** les éléments structurels (4, 5, 18) des deux structures de couplage coopérantes (3, 17) peuvent être amenés en prise par friction et par correspondance de forme.

3. Système d'implant osseux selon la revendication 1, **caractérisé en ce que** les ouvertures (4) sont formées par des trous borgnes à la surface du premier module d'implant osseux (1).

4. Système d'implant osseux selon l'une quelconque des revendications précédentes, **caractérisé en ce que** l'un module d'implant (10) est une augmentation et de préférence une augmentation acétabulaire et l'autre module d'impact (1) est de préférence une cupule acétabulaire.

5. Système d'implant osseux selon l'une quelconque des revendications précédentes, **caractérisé en ce qu'au** moins un, de préférence tous les modules d'implant osseux ont un corps de base avec une structure grillagée, qui devient ainsi à pores ouvertes au moins par zone partielle au niveau de sa surface, selon laquelle en plus des structures de couplage entre deux modules d'implant osseux couplés l'un à l'autre, un contact de friction des surfaces est réalisé.

6. Système d'implant osseux selon l'une quelconque des revendications précédentes, **caractérisé en ce que** les structures de couplage (3, 17) peuvent être mises à disposition d'un seul tenant de matière avec le module d'implant de préférence par un procédé de frittage laser ou séparées de préférence par des tiges ou des nattes grillagées séparées.

7. Système d'implant osseux selon l'une quelconque des revendications précédentes, **caractérisé en ce que** le matériau, à partir duquel les éléments structurels (5, 18) des structures de couplage (3, 17) sont réalisés, est différent du matériau de l'autre module d'implant de l'implant osseux (1, 10) ou a au moins une propriété de matériau différente de préférence une plus grande flexibilité.
